# EUROPEAN PATENT SPECIFICATION

(11) **EP 0 429 480 B1**
(45) Date of publication and mention of the grant of the patent: **22.04.1998**
(21) Application number: 89908739.9
(22) Date of filing: 10.07.1989
(51) Int. Cl.: A61K 31/56, C07J 1/00

(54) **NATRIURETIC HORMONE**
NATRIURETISCHES HORMON
HORMONE NATRIURETIQUE

(30) Priority: 11.07.1988 US 217458
(43) Date of publication of application: 05.06.1991
(73) Proprietor: Bricker, Neal S, Redlands, CA 92373 (US)
(72) Inventor: BRICKER, Neal, S., Redlands, CA 92373 (US); WECHTER, William, J., Redlands, CA 92373 (US)
(74) Representative: Brown, David Leslie
(86) International application number: US8902987
(87) International publication number: WO9000394

(56) References cited:
- US-A- 4 727 061
- THE AMERICAN JOURNAL OF PHYSIOLOGY vol. 224, no. 3 , 1973 , BETHESDA, US pages 651 - 658 M. POPOVTZER ET AL 'Acute Effect of Prednisolone on Renal Handling of Sodium'
- CHEMICAL ABSTRACTS, vol. 69, no. 21, 18 November 1968, Columbus, Ohio, US; abstract no. 83792, T. MATSUDA ET AL 'Clinical Studies on the Effect of Aldosterone and Aldosterone Antagonists on Water and Electrolyte Metabolism. I. Clinical Studies on the Effects of Aldosterone and Aldosterone Antagonists on Water and Electrolyte Metabolism in Renal Tubules' page 7830 ;column 1 ; & NAIKA HOKAN vol. 14, no. 1 , 1967 pages 19 - 33
- J. Clinical Investigation Vol 53(6), pp 1559-1667 (1974)

## Description

This invention relates to a compound having a natriuretic effect which can be used to increase sodium excretion in man or other animals.

### BACKGROUND OF THE INVENTION

The nature of the system controlling sodium excretion in man and other terrestrial mammals has been investigated for many years. Until the early 1960's, sodium excretion in mammals was believed to be controlled by changes in one of two factors: 1) glomerular filtration rate ("GFR"); and 2) mineralocorticoid hormone activity. In 1961, however, it was demonstrated by DeWardener et al., Clinical Science, 21:249-258 (1961), that an additional factor, or factors regulated sodium excretion. It was observed that increased sodium excretion (hereinafter referred to as "natriuresis") occurred in response to extracellular fluid volume expansion in dogs despite constant GFR and mineralocorticoid hormone activity.

Since the observations of DeWardener et al., considerable effort has been employed by researchers in the field to isolate and identify other factors involved in the regulation of sodium excretion. In the belief that there exists a principal modulator of sodium excretion, a number of researchers have pursued a substance referred to as the "natriuretic hormone." See, for example, Bricker, N.S., "The Control of Sodium Excretion With Normal and Reduced Nephron Populations: Pre-Eminence of Third Factor," Am. J. Med., 43:313 (1967); see also, Haber and Haupert, Hypertension, 4:315 (1987).

Prior to the present invention, natriuretic factors acting on the Na/KATPase pump had not been isolated in pure form, chemically defined, or synthesized in the laboratory. In fact, there is evidence that more than one biochemical compound may be responsible for observed effects. the inhibitors of active sodium transport inhibiting fractions studied by most investigators is a small molecule with a molecular weight of less than 500 Daltons (as indicated by ultra filtration). Some groups have reported that the compound could be a peptide, but recent studies have not supported the peptide nature of the factor.

Considerable research over the last several decades has focused on adverse effects of a high sodium diet (for example in hypertension) and on the renal retention of sodium in a number of diseases, including heart disease, liver failure and premenstrual syndromes. Diuretic agents are widely used today in an effort to prevent or reverse these pathologic states. However, most potent widely used diuretics not only increase sodium excretion but may also lead to undesirable loss of potassium. Unfortunately, the potassium supplements prescribed for replacement are generally unpalatable, expensive and difficult for patients to tolerate on a continuing basis.

There thus exists a need for a potent natriuretic compound which specifically augments sodium excretion but does not produce the loss of potassium. The present invention satisfies this need and provides related advantages as well.

### SUMMARY OF THE INVENTION

The present invention provides a substantially purified natriuretic compound, termed "Natriuretic Hormone," that increases sodium excretion without adversely affecting potassium excretion. The Natriuretic Hormone has a steroidal nucleus, a molecular weight of 360.4 and a molecular formula of C₂₁H₂₈O₅.

The present invention is defined in the appended claims.

According to claim 1, the natriuretic hormone comprises a compound characterized by its ability to increase sodium excretion in urine in mammals without a corresponding increase in potassium excretion, wherein said compound:
(i) has a molecular weight of about 360;
(ii) has a molecular formula of C₂₁H₂₈O₅;
(iii) has a steroidal nucleus;
(iv) is a white powder;
(v) exhibits a major ultraviolet absorbance peak at about 220 nm and a broad secondary peak at about 290 nm;
(vi) is highly resistant to temperature extremes, maintaining biological activity after over a year of exposure to -80°C or vigorous boiling;
(vii) is water soluble;
(viii) is polar;
(ix) exhibits a markedly lower biological activity when exposed to 5N HCl;
(x) inhibits transepithelial sodium transport
   (1) in an isolated urinary bladder of a toad;
   (2) in isolated skin of a frog;
   (3) in an isolated perfused cortical collecting tubule of a rabbit nephron;
(xi) produces an increase in sodium excretion in a normal Sprague-Dawley rat when fasted but allowed free access to water;
(xii) produces natriuresis in an unanaesthetized uremic rat; and
(xiii) inhibits sodium transport by MDBK cells;
or a modified form thereof in which esters of acid or hydroxylic functional groups are present without destroying said biological activity.

According to claim 2, the natriuretic hormone can be obtained from post-salt peak material separated by gel filtration from a concentrated mammalian sample containing the compound in an aqueous solvent, by a method comprising the steps of:
(a) isolating a biologically active fraction from said material by repeated reverse-phase high pressure chromatography on a resin column using pyridinium acetate/methanol as eluant;
(b) reacting said isolated biologically active fraction with a trimethylsilylating agent;
(c) applying said trimethylsilylated biologically active fraction through a gas chromatograph;
(d) recovering said gas chromatographed product having a retention time of about 200-700 seconds;
(e) subjecting said gas chromatographed product to hydrolysis with acid; and
(f) separating said hydrolyzed product whereby said compound is obtained.

In one aspect of the invention, the Natriuretic Hormone is obtained by lyophilizing and reconstituting at a reduced volume with deionized water the urine from uremic patients to obtain concentrated samples, separating the concentrated material by gel filtration, subjecting the post salt peak to reverse-phase high pressure liquid chromatography using a pyridinium acetate/methanol buffer. The biologically active material activity is eluted with about 45% methanol. The purified product of the HPLC is then trimethylsilylated and subjected to gas chromatography at elevated temperature. The single product of this chromatography is then hydrolyzed in acid solutions to yield after evaporation the Natriuretic Hormone.

In another aspect of the invention, the Natriuretic Hormone is used to increase the sodium excretion in a mammal.

### BRIEF DESCRIPTION OF THE DRAWING

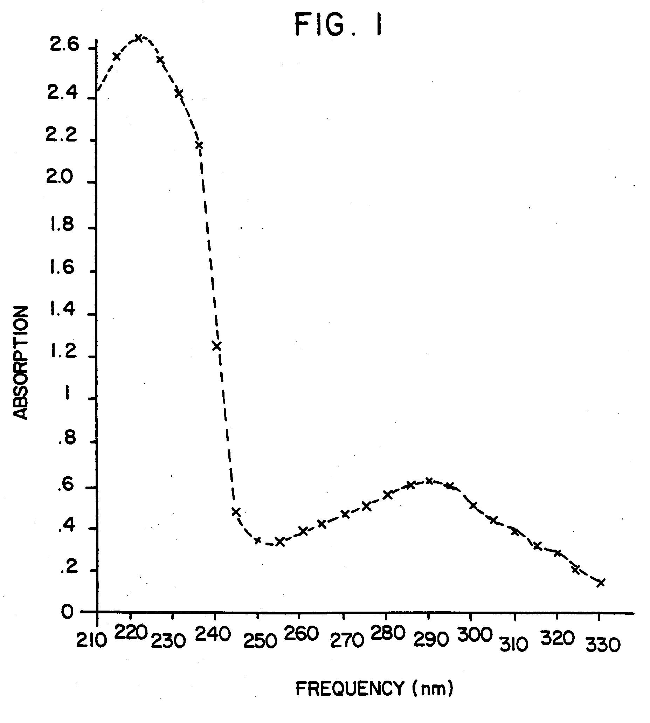

Figure 1 provides a representation of an ultraviolet absorption spectra of a purified active sample as described in Example I.

### DETAILED DESCRIPTION OF THE INVENTION

In the course of investigations leading to the present invention, a compound having natriuretic properties was isolated, in substantially pure form. The compound is termed the Natriuretic Hormone. This Natriuretic Hormone can be obtained from a variety of sources, including the blood and urine of mammals, preferably humans or dogs and homogenates of selected tissues such as the hypothalamus. Although the compound is present in normal human urine, the amount harvested per 24 hour urine collection is markedly greater in urine from Uremic individuals.

The Natriuretic Hormone has molecular weight of 360.4, a molecular formula of C₂₁H₂₈O₅ and a steroidal nucleus. Additional information relating to the physical properties of the Natriuretic Hormone is also available. It has been isolated as a white powder and exhibits a major ultraviolet absorbance peak at about 220 nm and a broad secondary peak at about 290 nm. The Natriuretic Hormone has been found to be highly resistant to temperature extremes, maintaining biologic activity as described below after over a year of exposure to minus 80°C or vigorous boiling. It is water soluble and has also been found to be soluble in certain organic solvents with high dielectric constants, indicating its polar nature. It is eluted from a Sephadex G-25 column employing ammonium acetate (NH₄OAc) buffer, pH 6.8, and appears after the salt peak.

The biological activity of the Natriuretic Hormone is markedly decreased by exposure to 5N HCl. It appears that the reaction taking place is due to interaction of acid with hydroxyl, carbonyl, or carboxyl groups on the steroidal nucleus, which are known to undergo elimination (for example, dehydration) , rearrangement (for example, reverse aldol), and/or hydrolysis (for example, lactone cleavage) reactions in appropriate circumstances.

Additionally, the Natriuretic Hormone can be further purified using a high pressure liquid chromatography (HPLC) column (reverse-phase HPLC using a C-18 resin column) employing a 0.2m pyridinium acetate buffer (pH 5.5)/methanol gradient, followed by repeated isocratic elution from the same column at a methanol concentration of about 40 to 50%, preferably 45%.

As used herein, the term "Natriuretic Hormone" refers to a compound which increases the rate of sodium excretion in at least one mammal upon administration by inhibiting Na-K-ATPase activity in the nephron. The native human Natriuretic Hormone has a molecular weight of 360.4, a molecular formula of C₂₁H₂₈O₅ and a steroid nucleus.

The term Natriuretic Hormone refers to both the native hormone and in vitro or in vivo modifications which retain natriuretic activity. It is understood that limited substitutions of acid, hydroxylic or carbonyl functional groups may be made without destroying the biological activity. Moreover, it will be recognized by those skilled in the arts of steroid chemistry and pharmaceutical preparations that many such derivatives can be made which are biologically and chemically equivalent to, or even more active than, the indicated compound in the present case such derivatives consist of esters of acid functions or esters of hydroxylic functions.

"Substantially pure," when used to describe the state of the Natriuretic Hormone, denotes the hormone essentially free of proteins, steroids, and other materials normally associated or occurring with Natriuretic Hormone in its native environment.

As used herein, the term "post salt peak" refers to material eluted from a G-25 Sephadex column which appears immediately after the sodium, potassium, urea and creatinine containing fractions and which has baseline conductivity and UV absorption at 280 nm.

The biological activity of the Natriuretic Hormone can be determined by a number of assay techniques which involve sodium transport in a wide variety of cell types and in a number of animal species. For example, transepithelial sodium transport is inhibited in the isolated urinary bladder of the toad, in the isolated skin of the frog and in the isolated perfused cortical collecting tubule of the rabbit nephron. In all three structures, inhibition of sodium transport occurs only if the Natriuretic Hormone is added to the blood side of the structure. In the isolated tubule preparation, the addition of surface produces a decrease in sodium efflux (lumen to bath), but there is no effect if it is added to the lumen surface. There is a simultaneous decrease in the transepithelial electrical potential difference (P.D.) with the lumen becoming less positive. In dose response studies, both sodium efflux and P.D. approach zero. As used herein, the term "biologically active" refers to material which results in greater than 20% inhibition of ⁸⁶Rb uptake as measured by the method of Example II.

In addition, the Natriuretic Hormone produces an increase in sodium excretion in the normal Sprague-Dawley rat when fasted but allowed free access to water. It also produces natriuresis in an unanesthetized uremic rat. When the hormone is infused directly into the renal artery of the normal rat kidney, natriuresis is moderate. When the equivalent dose is infused into the renal artery of the kidney of a uremic animal, the natriuretic response is markedly increased.

The Natriuretic Hormone inhibits sodium transport by MDBK cells, a cell line originally obtained from bovine renal tubules and maintained using cell culture techniques. Such a cell line is available from the American Type Culture Collection, 12301 Park Lawn Drive, Rockville, MD 20852, USA, where it is identified by the reference ATCC CRL6071 (certified cell line). Alternatively, other cell lines such as MDCK cells, derived from canine renal tubules (ATCC CCL34), could be used in the assay. The preferred method of assaying bioactivity according to the present invention, described in Example II below, employs a microassay which is based upon the ability of the natriuretic compound of the present invention to inhibit ⁸⁶Rb influx by MKBK cell grown in culture. Ouabain (10⁻⁵M) is used as the reference inhibitor. Natriuretic Hormone also inhibits sodium efflux and influx (in short-term measurements) as wells as ⁸⁶Rb by the MDBK cell line in culture.

The Natriuretic Hormone of the invention can be used in a number of clinical and diagnostic contexts. Clinical indications include edematous conditions, such as congestive heart failure, cirrhosis of the liver accompanied by edema or ascites, nephrotic syndrome, and hypertensive conditions. Because the compound of the invention is resistant to inactivation under acidic or alkaline conditions, oral ingestion is possible and preferred. However, administration by other routes, such as intravenous, intramuscular, transdermal and the like, is also effective.

The amount administered at any one time will increase sodium excretion sufficiently to provide a beneficial clinical result. Later doses can be adjusted in accordance with clinical effects of the initial dose. A typical initial dose would be from about 1-1000 mg in an average human, preferably 10-250 mg, and more preferably 25-100 mg. The total daily dose can consist of a single individual dose or multiple doses given at intervals.

Pharmaceutical preparations can include, in addition to the active compound, various inert carriers and/or other inactive components such as moistening agents, flavors, binding agents, and extenders, as well as other compounds having pharmacological activities, such as other diuretics which increase the distal delivery of sodium (i.e., acetazolamide).

The pharmaceutical compositions can take the form of tablets, capsules, injectable solutions and suspensions, oral solutions, and other formulations intended for pharmaceutical use. For example, a composition intended for use in a tablet could contain 25 mg active material, calcium stearate, calcium sulfate, microcrystalline cellulose, peppermint oil, polysorbate 80, povidone, and pregelatinized starch.

In addition to use in humans, the natriuretic compound of the invention can be used for similar veterinary purposes in domesticated animals, particularly pets with sodium retaining diseases and high blood pressure.

The following examples are intended to illustrate but not limit the invention. While they are typical of those that might be used, other procedures known to those skilled in the art may be alternatively employed.

### EXAMPLE I

### Isolation of Natriuretic Hormone

Twenty four hour urine samples were collected over periods of one to ten days from non-dialyzed patients with serum creatinine concentrations of over 8 mg/dl and/or a serum creatinine clearance of less than 20-25 ml/minute. Notation was made of the volume of urine collected each day, and of all medications taken by the patient. Each 24 hour urine collection was lyophilized to a sludge under reduced pressure and temperature and reconstituted in 100 ml of deionized water. The preparation was then centrifuged at 3000 rpm at 4°C and filtered through fluted filter papers, (Whatmann #1).

In brief, 25 ml aliquots of the concentrated urine samples, equivalent to 6 hour samples of original urine, were applied to individual 2.5 x 95-cm columns packed with Sephadex G-25 (fine grade, Pharmacia Fine Chemicals, Inc., Piscataway, NJ). Elution was carried out at 4°C by gravity at a rate of 55 to 65 ml/h with a solution of 10 mM ammonium acetate at pH 6.8. The effluent solution was collected overnight in 18 x 150-nm glass tubes (12 ml) using an automated fraction collector (model 7000 Ultrorac, LKB Producer AB, Stockholm, Sweden). On the basis of the ultraviolet absorption at 280 nm (LKB Uvicord) and of the electrical conductivity tracings (model 5300B Conductolyzer with 5312B Conductivity cell, LKB Producter AB), the effluent solution and the contents of the tubes were pooled into several different fractions. The fractions containing high molecular weight compounds (for example, proteins), sodium, potassium, urea, and creatinine were discarded. Natriuretic activity was present only in the fractions appearing immediately after the salt peak. Only this portion of the effluent solution was then routinely prepared by pooling the contents of 10 consecutive tubes (120 ml total) starting with the tube in which the specific conductance of the eluate had returned to base-line values and lyophilizing the material to dryness overnight in a glass container.

The dried eluate was then dissolved in 2.5 ml of 80% 0.2M pyridinium acetate (pH 5.5)/20% methanol transferred into a screw cap glass or polyethylene vial, and stored at -80°C. Each milliliter of this solution was equivalent to the volume of original urine excreted in approximately 2 hours. Storage of the concentrated urine samples or of the final fractions for periods of up to several months was found to have no influence on the natriuretic activity of the material.

The standard fractions subjected to assay, whether obtained from uremic patients or normal subjects, typically contained less than 10 meq/liter of sodium and 1 meq/liter of potassium. Differences were not observed between fractions from uremic patients and normal subjects for ammonium, urea, and protein concentrations. The mean values were: ammonium, 24.4 ± 3.7 meq/liter; urea, 15.8 ± 2.6 mg/100 ml; and protein, 2.0 ± 0.6 mg/ml.

Biologically active fractions, as determined by the method of Example II, of post salt peak from the G-25 eluate were applied to a reverse phase chromatography column RP-C-18 (Brownlee Labs, Licrosorb RP-18, 10 microns, Serial 8771) and eluted with 80% 0.2M pyridinium acetate (pH 5.5)/20% methanol for 10 minutes at about 1 ml per minute followed by a gradient to 50% 0.2 M pyridinium acetate (pH 5.5)/50% methanol over a 60 minute period. Fractions were collected at one minute intervals. Alternatively, Beckman Ultrasphere ODS (C-18), (Beckman Instruments, Brea, CA) 5 microns, can be used, although the time at which the desired compound is eluted will vary and must be confirmed by the in vivo and in vitro assays described herein. The desired compound, which eluted at about 45 minutes plus or minus 5 minutes, was detected by fluorescence transmission (Aminco Fluoro Monitor, American Instruments Co., Silver Spring, MD, employing glass filters: excitation 310-410 nm and emission 475-650 nm. Individual tubes were evaporated to dryness employing a "Speed Vacuum" (Savant Instruments, Farmingday, NY), redissolved in deionized water and then tested for biological activity by the method of Example II. Biologically active fractions were combined and re-chromatographed on the same column by isocratic elution using 56% 0.2M pyridinium acetate (pH 5.5)/44% methanol. In this way an apparently homogeneous solid was isolated after evaporation of appropriate fractions chosen on the basis of their ability to inhibit radioactive rubidium transport as described by the Example II assay. The in vivo activity of this material as well as that from previous steps in the purification was confirmed as natriuretic in the rat as described by Bricker et al., J. Clin. Invest., 53:1559-1567 (1974), which is incorporated herein by reference.

The ultraviolet absorption spectrum of this material exhibited maxima at approximately 220 and 290 nm. The material exhibited intrinsic fluorescence in a Beckman Fluorometer, Beckman Instruments, Brea, CA.

The biologically active material was then reacted with a large excess of 1:1 pyridine:BSTFA (bistrimethylsilyl trifluoroacetamide) at 50°C for 30 minutes. The resulting mixture was then applied to a gas chromatograph, using a 15 meter fused silica capillary column (J&W Scientific DB-5-30W) employing a high resolution E-I mass spectrometer as a detector. The retention time of the single product was 200 to 700 seconds. No other products were detected. The trimethylsilyl groups were hydrolyzed by treatment with 1N hydrochloric acid for one hour giving, after lyophilization, a substantially pure white solid, the Natriuretic Hormone.

### EXAMPLE II

### Microassay For Biological Activity

The natriuretic activity of the Natriuretic Hormone can be ascertained by its effect on the uptake of ⁸⁶Rb by MDBK (Malbin Darby Bovine Kidney Cell) cultures. Briefly, uptake by the cells of ⁸⁶Rb (which behaves biologically as potassium) is inhibited by factors which inhibit the activity of the enzyme, Na-K-ATPase. Ouabain is a known inhibitor of this enzyme an the addition of ouabain to an MDBK cell preparation containing ⁸⁶Rb can result in as much as 90% inhibition of ⁸⁶Rb intake by the cells.

Approximately 200,000 MDBK cells were plated in trays containing 12 2mm² wells, and grown for four days at 37°C in Dulbecco's modified Eagle media (DMEM) (Gibco #430-2100), to which was added (per liter) 3.7g sodium bicarbonate, 5 mg phenol red, 10% fetal calf serum 10⁶ units penicillin-G sulfate, 0.1g equivalent base streptomycin sulfate, 7.34 mg polymyxin-B sulfate, and 3.4 mg fungizone and maintained in an atmosphere of 5% CO₂, 95% air. The cells became confluent and present at a concentration of approximately 10⁶ cells per well. To perform the assay, the cells were preincubated for thirty minutes with the test material 50-75 µl in volume which represents at least 2-5 minutes of original urine, in fortified DMEM (500 µl) at 37°C. After this preincubation, approximately 5 x 10⁵ cpm ⁸⁶Rb and an additional 50-75 µl of test material were added to each well and the cells incubated at 37°C for an additional 15 minutes. The reaction was stopped with 1 ml of cold PBS and washed twice with 0.5 ml of cold PBS. The washes were discarded. 0.5 ml of 5% TCA was added to each well and incubated for 10 minutes at 37°C, to release radioactivity inside the cells. The supernatants from each well were then transferred to a microfuge tube (1 ml). A 100 µl aliquot (in duplicate) was transferred to scintillation vials containing scintillation fluid (Ready Safe for aqueous samples; Beckman Instruments, Brea, CA). The ⁸⁶Rb activity was counted in a scintillation counter (Beckman LS 3-801). The cells were then washed with PBS and digested overnight at 37°C in 500 µl 0.1 N NaOH. The next day, the hydrolyzed cells were analyzed for protein by the micro Coomassie method (Chiapelli, F. et al., Analytical Bioch., 94:160, 1974), which is incorporated herein by reference. In each set of assays, PBS and 10⁻⁵ M Ouabain were used for negative and positive controls, respectively. Data were recorded as cpm/mg cell protein. Fractions which inhibited ⁸⁶Rb uptake by more than 20% were considered active. Most active samples inhibited ⁸⁶Rb uptake by a substantially greater percentage. Typical results are shown in Table I.

Although the invention has been described with reference to the presently preferred embodiment, it should be understood that various modifications can be made. Accordingly, the invention is limited only by the following claims.

**TABLE I**

| Each sample run in duplicate and averaged. | | |
|---|---|---|
| Control +PBS | 79,692 | 0% |
| | 69,058 | |
| Ouabain ¹⁰⁻⁵ | 17,649 | 77.7% |
| | 15,611 | |
| G-25 "Post Salt" Natriuretic Activity (10 µL) | 45,592 | 41.4% |
| | 41,596 | |
| (50 µL) | 32,336 | 58.0% |
| | 20,803 | |

## Claims

1. A substantially purified natriuretic hormone comprising a compound characterized by its ability to increase sodium excretion in urine in mammals without a corresponding increase in potassium excretion, wherein said compound:
(i) has a molecular weight of about 360;
(ii) has a molecular formula of C₂₁H₂₈O₅;
(iii) has a steroidal nucleus;
(iv) is a white powder;
(v) exhibits a major ultraviolet absorbance peak at about 220 nm and a broad secondary peak at about 290 nm;
(vi) is highly resistant to temperature extremes, maintaining biological activity after over a year of exposure to -80°C or vigorous boiling;
(vii) is water soluble;
(viii) is polar;
(ix) exhibits a markedly lower biological activity when exposed to 5N HCl;
(x) inhibits transepithelial sodium transport
(1) in an isolated urinary bladder of a toad;
(2) in isolated skin of a frog;
(3) in an isolated perfused cortical collecting tubule of a rabbit nephron;
(xi) produces an increase in sodium excretion in a normal Sprague-Dawley rat when fasted but allowed free access to water;
(xii) produces natriuresis in an unanaesthetized uremic rat; and
(xiii) inhibits sodium transport by MDBK cells;
or a modified form thereof in which esters of acid or hydroxylic functional groups are present without destroying said biological activity.

2. A method of obtaining a substantially purified natriuretic hormone compound according to claim 1 from post-salt peak material separated by gel filtration from a concentrated mammalian sample containing said compound in an aqueous solvent, comprising the steps of:
(a) isolating a biologically active fraction from said material by repeated reverse-phase high pressure chromatography on a resin column using pyridinium acetate/methanol as eluant;
(b) reacting said isolated biologically active fraction with a trimethylsilylating agent;
(c) applying said trimethylsilylated biologically active fraction through a gas chromatograph;
(d) recovering said gas chromatographed product having a retention time of about 200-700 seconds;
(e) subjecting said gas chromatographed product to hydrolysis with acid; and
(f) separating said hydrolyzed product whereby said compound is obtained.

3. A method of obtaining substantially purified natriuretic hormone according to claim 2, wherein the post-salt peak material is prepared by a method comprising the steps of:
(a) pre-concentrating a mammalian sample containing said compound in an aqueous solvent by removing said solvent from said mammalian sample containing said compound;
(b) reconstituting said pre-concentrated sample to a reduced volume with deionized water; and
(c) applying said reconstituted sample to gel filtration through a Sephadex G-25 column using a solution of ammonium acetate as an eluant and retaining only material appearing immediately after a salt peak to obtain a post-salt peak material.

4. A method of obtaining substantially purified natriuretic hormone according to claim 3, wherein further said pre-concentration is by lyophilization.

5. A method of obtaining substantially purified natriuretic hormone according to claim 3 or 4, wherein the eluant is a 10mM solution of ammonium acetate at pH 6.8.

6. A method of obtaining substantially purified natriuretic hormone according to any one of claims 2 to 5, wherein further said mammalian sample is selected from the group consisting of blood, urine and hypothalamus tissue.

7. A method of obtaining substantially purified natriuretic hormone according to any one of claims 2 to 6, wherein further said trimethylsilylating agent is 1:1 pyridine:bistrimethylsilyl trifluorocetamide.

8. A method of obtaining substantially purified natriuretic hormone according to any one of claims 2 to 7, wherein further said hydrolysis with acid is conducted with 1N hydrochloric acid.

9. A substantially purified natriuretic hormone according to claim 1, obtainable by a method according to any one of claims 2 to 8.

10. A natriuretic hormone according to either of claims 1 and 9, for use as a medicament.

11. A natriuretic hormone according either of claims 1 and 9, for use as a medicament for treating mammals with edematous conditions or hypertension.

12. Use of a natriuretic hormone according to either of claims 1 and 9 for the preparation of a medicament for treating edematous conditions or hypertension in a mammal.

13. A pharmaceutical composition useful for treating mammals with edematous conditions or hypertension, comprising an amount of a natriuretic hormone compound according to either of claims 1 and 9 effective to treat said edematous conditions or hypertension and a pharmaceutically acceptable carrier.

14. A pharmaceutical composition according to claim 13, wherein said compound is present in an amount of about 1-1000mg.

15. A pharmaceutical composition according to claim 13 or 14, wherein said compound is present in an amount of about 10-250mg.

16. A pharmaceutical composition according to any one of claims 13 to 15, wherein said compound is present in an amount of about 25-100mg.

17. A pharmaceutical composition according to any one of claims 13 to 16, wherein said carrier comprises moistening agents, flavors, binding agents and extenders.

18. A pharmaceutical composition according to any one of claims 13 to 17, wherein said composition is in a form selected from a group consisting of tablets, capsules, injectable solutions, suspensions and oral solutions.

19. A pharmaceutical composition according to claim 18, wherein said tablets comprise about 25mg of said compound, calcium stearate, calcium sulfate, microcrystalline cellulose, peppermint oil, polysorbate 80, povidone and pregelatinized starch.

## Patentansprüche

1. Im wesentlichen gereinigtes natriuretisches Hormon, das eine Substanz aufweist, welche dadurch gekennzeichnet ist, daß sie in der Lage ist, die Ausscheidung von Natrium im Urin von Säugetieren zu steigern ohne einen entsprechenden Anstieg der Ausscheidung von Kalium zu bewirken, wobei diese Substanz:
(i) ein Molekulargewicht von etwa 360 aufweist;
(ii) eine Summenformel von C₂₁H₂₈O₅ aufweist;
(iii) einen steroiden Kern aufweist;
(iv) ein weißes Pulver ist;
(v) einen ultravioletten Haupt-Absorptionspeak bei etwa 220 nm und einen breiten sekundären Peak bei etwa 290 nm aufweist;
(vi) hochresistent gegen extreme Temperaturen ist und biologische Aktivität nach mehr als 1 Jahr bei
-80 °C oder kräftigem Kochen behält; (vii) wasserlöslich ist;
(viii) polar ist;
(ix) eine deutlich geringere biologische Aktivität aufweist, wenn sie einer 5N HCl ausgesetzt wird;
(x) den transepithelialen Natriumtransport
(1) in einer isolierten Harnblase einer Kröte;
(2) in isolierter Haut eines Frosches;
(3) in einem isolierten, durchströmten, kortikalen Sammeltubulus eines Nephrons von einem Kaninchen;
inhibiert;
(xi) einen Anstieg in der Ausscheidung von Natrium in gesunden Sprague-Dawley-Ratten erzeugt, wenn diese fasten jedoch freien Zugang zu Wasser haben;
(xii) Natriurese in nicht-anästhesierten urämischen Ratten hervorruft; und
(xiii) den Natrium-Transport der MDBK-Zellen hemmt;
oder eine modifizierte Form davon, in der Ester von Säuren oder funktionellen Hydroxylgruppen vorliegen ohne die biologische Aktivität zu zerstören.

2. Verfahren zur Gewinnung einer im wesentlichen gereinigten natriuretischen Hormon-Substanz nach Anspruch 1 aus Material nach dem Salz-Peak, das mittels Gelfiltration von einer aufkonzentrierten Probe von einem Säugetier, welche diese Substanz in einem wäßrigen Lösungsmittel enthält, abgetrennt wurde, dadurch gekennzeichnet, daß man:
(a) eine biologisch aktive Fraktion des Materials mittels wiederholter Umkehrphasen-Hochdruck-Chromatographie über eine Harz-Säule unter Verwendung von Pyridinium Acetat/Methanol als Elutionsmittel isoliert;
(b) die isolierte, biologisch aktive Fraktion mit einem trimethylsilylierenden Agens umsetzt;
(c) die trimethylsilylierte, biologisch aktive Fraktion durch einen Gaschromatographen schickt;
(d) das Gas-chromatographierte Produkt mit einer Retentionszeit von etwa 200-700 Sekunden gewinnt;
(e) das Gas-chromatographierte Produkt mittels Säure einer Hydrolyse unterwirft; und
(f) das hydrolysierte Produkt auftrennt, wobei die Substanz erhalten wird.

3. Verfahren zur Gewinnung eines im wesentlichen gereinigten, natriuretischen Hormons nach Anspruch 2, dadurch gekennzeichnet, daß das Material nach dem Salz-Peak durch ein Verfahren hergestellt wird, bei dem man:
(a) eine Probe von einem Säugetier, die die Substanz in einem wäßrigen Lösungsmittel enthält, vorkonzentriert, indem man das wäßrige Lösungsmittel aus der Säugetierprobe, die die Substanz enthält, entfernt;
(b) die vorkonzentrierte Probe mit einem reduzierten Volumen an deionisiertem Wasser rekonstituiert; und
(c) die rekonstituierte Probe einer Gelfiltration über eine Sephadex G-25 Säule unter Verwendung einer Ammoniumacetat-Lösung als Elutionsmittel unterwirft und lediglich das Material gewinnt, welches unmittelbar nach dem Salz-Peak erscheint, um das Material nach dem Salz-Peak zu erhalten.

4. Verfahren zur Gewinnung eines im wesentlichen gereinigten, natriuretischen Hormons nach Anspruch 3, dadurch gekennzeichnet, daß man ferner die Vorkonzentration mittels Lyophilisation durchführt.

5. Verfahren zur Gewinnung eines im wesentlichen gereinigten, natriuretischen Hormons nach den Ansprüchen 3 oder 4, dadurch gekennzeichnet, daß ferner das Elutionsmittel eine 10 mM Ammoniumacetat-Lösung mit einem pH-Wert von 6,8 ist.

6. Verfahren zur Gewinnung eines im wesentlichen gereinigten, natriuretizchen Hormons nach einem der Ansprüche 2 bis 5, dadurch gekennzeichnet, daß ferner die Säugetierprobe aus der Gruppe bestehend aus Blut, Urin und Hypothalamusgewebe ausgewählt ist.

7. Verfahren zur Gewinnung eines im wesentlichen gereinigten, natriuretischen Hormons nach einem der Ansprüche 2 bis 6, dadurch gekennzeichnet, daß ferner das Agens zur Trimethylsilylierung aus 1 : 1 Pyridin : Bistrimethylsilyltrifluoracetamid besteht.

8. Verfahren zur Gewinnung eines im wesentlichen gereinigten, natriuretischen Hormons nach einem der Ansprüche 2 bis 7, dadurch gekennzeichnet, daß man ferner die Säure-Hydrolyse mit einer 1N Salzsäure durchführt.

9. Im wesentlichen gereinigtes natliuretisches Hormon nach Anspruch 1, erhältlich durch ein Verfahren nach einem der Ansprüche 2 bis 8.

10. Natriuretisches Hormon nach einem der Ansprüche 1 und 9 zur Verwendung als Arzneimittel.

11. Natrirnretisches Hormon nach einem der Ansprüche 1 und 9 zur Verwendung als Arzneimittel zur Behandlung ödematöser Zustände oder von Hochdruck.

12. Verwendung eines natriuretischen Hormons nach einem der Ansprüche 1 und 9 zur Herstellung eines Arzneimittels zur Behandlung ödematöser Zustände oder von Hochdruck in einem Säugetier.

13. Pharmazeutische Zusammensetzung zur Behandlung von Säugetieren mit ödematösen Zuständen oder Hochdruck, dadurch gekennzeichnet, daß die Zusammensetzung eine ausreichende Menge einer natriuretischen Hormon-Substanz nach einem der Ansprüche 1 und 9 aufweist, um die ödematösen Zustände oder den Hochdruck zu behandeln, und ein pharmazeutisch akzeptablen Trägerstoff.

14. Pharmazeutische Zusammensetzung nach Anspruch 13, dadurch gekennzeichnet, daß die Substanz in einer Menge von etwa 1-1000 mg vorliegt.

15. Pharmazeutische Zusammensetzung nach den Ansprüchen 13 oder 14, dadurch gekennzeichnet, daß die Substanz in einer Menge von etwa 10-250 mg vorliegt.

16. Pharmazeutische Zusammensetzung nach einem der Ansprüche 13 bis 15, dadurch gekennzeichnet, daß die Substanz in einer Menge von etwa 25-100 mg vorliegt.

17. Pharmazeutische Zusammensetzung nach einem der Ansprüche 13 bis 16, dadurch gekennzeichnet, daß der Trägerstoff Befeuchtungsmittel, Geschmacksstoffe, Bindemittel und Verdünnungsmittel umfaßt.

18. Pharmazeutische Zusammensetzung nach einem der Ansprüche 13 bis 17, dadurch gekennzeichnet, daß die Zusammensetzung eine Form aufweist, die aus der Gruppe bestehend aus Tabletten, Kapseln, Injektionslösungen, Suspension und oral verabreichbaren Lösungen ausgewählt ist.

19. Pharmazeutische Zusammensetzung nach Anspruch 18, dadurch gekennzeichnet, daß die Tabletten etwa 25 mg der Substanz, Calciumstearat, Calciumsulfat, mikrokristalline Cellulose, Pfefferminzöl, Polysorbat 80, Povidon und vorgelatinierte Stärke umfassen enthalten.

## Revendications

1. Hormone natriurétique sensiblement purifiée comprenant un composé caractérisé par son aptitude à augmenter l'excrétion de sodium dans l'urine chez les mammifères sans une augmentation correspondante de l'excrétion de potassium, dans laquelle ce composé :
(i) a un poids moléculaire de 360 environ;
(ii) a une formule moléculaire de C₂₁H₂₈O₅;
(iii) a un noyau stéroïdien;
(iv) est une poudre blanche;
(v) présente un pic principal d'absorption ultraviolette à 220 nm environ et un pic secondaire large à 290 nm environ;
(vi) est extrêmement résistant à des extrêmes de température, conservant une activité biologique après plus d'un an d'exposition à -80°C ou une ébullition vigoureuse;
(vii) est soluble dans l'eau;
(viii) est polaire;
(ix) présente une activité biologique nettement plus faible quand il est exposé à HCl 5N;
(x) inhibe le transport transépithélial de sodium
(1) dans une vessie urinaire isolée d'un crapaud;
(2) dans de la peau isolée d'une grenouille;
(3) dans un tube collecteur cortical perfusé isolé d'un néphron de lapin;
(xi) produit une augmentation de l'excrétion de sodium chez un rat normal de Sprague-Dawley à jeun mais pouvant accéder librement à l'eau;
(xii) produit la natriurèse chez un rat urémique non anesthésié; et
(xiii) inhibe le transport de sodium par les cellules MDBK;
ou une forme modifiée de ce composé dans laquelle des esters de groupes fonctionnels acides ou hydroxyles sont présents sans détruire l'activité biologique.

2. Méthode pour obtenir un composé à base d'hormone natriurétique sensiblement purifiée suivant la revendication 1 à partir d'une matière au pic postérieur aux sels séparée par filtration sur gel d'un échantillon concentré de mammifère contenant le composé dans un solvant aqueux, comprenant les stades consistant à :
(a) isoler une fraction biologiquement active de cette matière par chromatographie répétée à haute pression en phase inverse sur une colonne de résine en utilisant un mélange d'acétate de pyridinium et de méthanol comme éluant;
(b) mettre la fraction biologiquement active isolée à réagir sur un agent de triméthylsilylation;
(c) soumettre la fraction biologiquement active triméthylsilylée à un chromatographe en phase gazeuse;
(d) récupérer le produit chromatographié en phase gazeuse ayant un temps de rétention de 200 à 700 secondes environ;
(e) soumettre le produit chromatographié en phase gazeuse à une hydrolyse par un acide; et
(f) séparer le produit hydrolysé de manière à obtenir le composé.

3. Méthode pour obtenir une hormone natriurétique sensiblement purifiée suivant la revendication 2, dans laquelle la matière au pic postérieur aux sels est préparée par une méthode comprenant les stades consistant à :
(a) préconcentrer un échantillon de mammifère contenant le composé dans un solvant aqueux en éliminant le solvant de l'échantillon de mammifère contenant le composé;
(b) reconstituer l'échantillon préconcentré à un volume réduit avec de l'eau désionisée; et
(c) soumettre l'échantillon reconstitué à une filtration sur gel dans une colonne Sephadex G-25 en utilisant une solution d'acétate d'ammonium comme éluant et en gardant seulement la matière apparaissant immédiatement après un pic postérieur aux sels, pour obtenir une matière au pic postérieur aux sels.

4. Méthode pour obtenir une hormone natriurétique sensiblement purifiée suivant la revendication 3, dans laquelle la préconcentration se fait par lyophilisation;

5. Méthode pour obtenir une hormone natriurétique sensiblement purifiée suivant la revendication 3 ou 4, dans laquelle l'éluant est une solution 10mM d'acétate d'ammonium au pH 6,8.

6. Méthode pour obtenir une hormone natriurétique sensiblement purifiée suivant l'une quelconque des revendications 2 à 5, dans laquelle l'échantillon de mammifère est choisi dans le groupe consistant en sang, en urine et en tissu hypothalamique.

7. Méthode pour obtenir une hormone natriurétique sensiblement purifiée suivant l'une quelconque des revendications 2 à 6, dans laquelle l'agent de triméthylsilylation est le trifluoroacétamide de pyridine:bistriméthylsilyle 1:1.

8. Méthode pour obtenir une hormone natriurétique sensiblement purifiée suivant l'une quelconque des revendications 2 à 7, dans laquelle l'hydrolyse par un acide est effectuée par l'acide chlorhydrique 1N.

9. Hormone natriurétique sensiblement purifiée suivant la revendication 1, pouvant être obtenue par une méthode suivant l'une quelconque des revendications 2 à 8.

10. Hormone natriurétique suivant l'une quelconque des revendications 1 et 9, destinée à être utilisée comme un médicament.

11. Hormone natriurétique suivant l'une quelconque des revendications 1 et 9, destinée à être utilisée comme un médicament pour traiter des mammifères ayant un état oedémateux ou une hypertension.

12. Utilisation d'une hormone natriurétique suivant l'une quelconque des revendications 1 et 9 pour la préparation d'un médicament pour traiter un état oedémateux ou une hypertension chez un mammifère.

13. Composition pharmaceutique utile pour traiter des mammifères ayant un état oedémateux ou une hypertension, comprenant une quantité d'un composé à base d'hormone natriurétique suivant l'une quelconque des revendications 1 et 9 efficace pour traiter cet état oedémateux ou cette hypertension et un excipient pharmaceutiquement acceptable.

14. Composition pharmaceutique suivant la revendication 13, dans laquelle le compose est présent en une quantité de 1 à 1000 mg environ.

15. Composition pharmaceutique suivant la revendication 13 ou 14, dans laquelle le composé est présent en une quantité de 10 à 250 mg environ.

16. Composition pharmaceutique suivant l'une quelconque des revendications 13 à 15, dans laquelle le composé est présent en une quantité de 25 à 100 mg environ.

17. Composition pharmaceutique suivant l'une quelconque des revendications 13 à 16, dans laquelle l'excipient comprend des agents humectants, des arômes, des liants et des charges.

18. Composition pharmaceutique suivant l'une quelconque des revendications 13 à 17, dans laquelle la composition est sous une forme choisie dans un groupe consistant en comprimés, capsules, solutions injectables, suspensions et solutions orales.

19. Composition pharmaceutique suivant la revendication 18, dans laquelle les comprimés comprennent 25 mg environ du composé, du stéarate de calcium, du sulfate de calcium, de la cellulose microcristalline, de l'essence de menthe poivrée, du polysorbate 80, de la povidone et de l'amidon prégélatinisé.
